# EUROPEAN PATENT SPECIFICATION

(11) **EP 3 936 522 B1**
(45) Date of publication and mention of the grant of the patent: **08.01.2025**
(21) Application number: 20769656.8
(22) Date of filing: 06.03.2020
(51) Int. Cl.: C07K 16/14, G01N 33/53, G01N 33/543, C12P 21/08, G01N 33/569, G01N 33/579

(54) **METHOD FOR IMMUNOLOGIC ANALYSIS OF ß-D-GLUCAN IN BIOLOGICAL SAMPLE, AND ß-D-GLUCAN ANALYSIS KIT**
VERFAHREN ZUR IMMUNOLOGISCHEN ANALYSE VON SS-D-GLUCAN IN EINER BIOLOGISCHEN PROBE UND SS-D-GLUCAN-ANALYSEKIT
PROCÉDÉ D'ANALYSE IMMUNOLOGIQUE DE ß-D-GLUCANE DANS UN ÉCHANTILLON BIOLOGIQUE ET KIT D'ANALYSE DE ß-D-GLUCANE

(30) Priority: 08.03.2019 JP 2019042753
(43) Date of publication of application: 12.01.2022
(73) Proprietor: Sekisui Medical Co., Ltd., Tokyo 103-0027 (JP)
(72) Inventor: SUNAMURA Ei-ichiro, Tokyo 103-0027 (JP); MIYAZAKI Osamu, Tokyo 103-0027 (JP); YOTANI Takuya, Tokyo 103-0027 (JP); KITAHARA Shinichiro, Tokyo 103-0027 (JP); IWASAKI Manami, Tokyo 103-0027 (JP)
(74) Representative: Hoffmann Eitle
(86) International application number: PCT/JP2020/009569
(87) International publication number: WO 2020/184409

(56) References cited:
- WO-A1-2020/022467
- WO-A1-99/31510
- JP-A- 2008 273 917
- JP-A- H04 346 791
- HIROSI TAMURA ET AL: "Plasma (1-3)-beta-D-glucan assay and immunohistochemical staining of (1-3)-beta-D-glucan in the fungal cell walls using a novel horseshoe crab protein (T-GBP) the specifically binds to (1-3)-beta-D-glucan", JOURNAL OF CLINICAL LABORATORY ANALYSIS, NEW YORK, NY, US, vol. 11, 1 January 1997 (1997-01-01), pages 104 - 109, XP002970551, ISSN: 0887-8013, DOI: 10.1002/(SICI)1098-2825(1997)11:2<104::AID-JCLA6>3.0.CO;2-B
- MEIKLE P J ET AL: "THE LOCATION OF 1-3-BETA GLUCANS IN THE WALLS OF POLLEN TUBES OF NICOTIANA-ALATA USING A 1-3-BETA GLUCAN-SPECIFIC MONOCLONAL ANTIBODY", PLANTA, SPRINGER BERLIN HEIDELBERG, BERLIN/HEIDELBERG, vol. 185, no. 1, 1 January 1991 (1991-01-01), pages 1 - 08, XP009063437, ISSN: 0032-0935, DOI: 10.1007/BF00194507
- SANDER I ET AL: "Development of a two-site enzyme immunoassay based on monoclonal antibodies to measure airborne exposure to (1->3)-@b-d-glucan", JOURNAL OF IMMUNOLOGICAL METHODS, ELSEVIER SCIENCE PUBLISHERS B.V.,AMSTERDAM, NL, vol. 337, no. 1, 20 August 2008 (2008-08-20), pages 55 - 62, XP023172879, ISSN: 0022-1759, [retrieved on 20080610], DOI: 10.1016/J.JIM.2008.05.010

## Description

The present invention relates to an immunoassay method for β-D-glucan (hereinafter sometimes abbreviated as BG) in a biological sample. The present invention also relates to an analysis kit forβ-D-glucan for assaying BG in a biological sample. The present invention also relates to a monoclonal antibodies binding to laminaritetraose.

### BACKGROUND ART

B-D-glucan is a glucose polymer in which multiple glucose molecules are bound by β-bonds. A carbon atom at the 1-position of glucose can bind to each of 5 carbons of the other glucose, i.e., carbons at the 1-position, the 2-position, the 3-position, the 4-position, and the 6-position. It has been reported that a bonding combination of the 1-position and 3-position (1->3), the 1-position and 4-position (1→4), or the 1-position and 6-position frequently occurs in natural β-D-glucan.

B-D-glucan is a cell-wall constituent component of fungi and is a characteristic substance not found in other microorganisms such as bacteria. Due to this feature, β-D-glucan analysis has been used in a test for deep mycosis. Deep mycosis is a type of opportunistic infection with which a patient with immune dysfunction due to weakened resistance, and the patient falls into an extremely critical condition. Examples of typical causative fungi of deep-mycosis include the genera Candida and Aspergillus. Since the presence of BG is common to the cell walls of these causative fungi, measurement of BG in body fluid has been used for auxiliary diagnosis of deep mycosis infections. The structure of β-glucan in clinical specimens is considered to be different depending on the causative fungi of deep mycosis, and a particularly large number of these causative fungi considered to have the cell walls composed of (1→3)-β-D-glucan (hereinafter sometimes abbreviated as (1->3)BG) and (1→3)(1→6)-β-D-glucan (hereinafter sometimes abbreviated as (1→3)(1→6)BG).

Currently, a Limulus amebocyte lysate reagent utilizing a protective reaction of horseshoe crab to BG is used for examination of deep mycosis (Patent Documents 1 and 2). This Limulus amebocyte lysate reagent is approved as an in-vitro diagnostic. However, a method using this Limulus amebocyte lysate reagent requires blood of horseshoe crab, which is a natural resource, and is therefore costly to maintain a certain quality in addition to causing a concern of resource depletion. Another drawback of this method is that dispersion of the measurement values tends to occur since multiple steps with manual operation are required for this technique. Horseshoe crab protein (T-GBP) that specifically binds to (1-3) -beta-D-glucan has also been used in immunohistochemical staining and assaying of BG in the fungal cell walls (Non-Patent Document 3).

In recent years, to eliminate the drawbacks of the Limulus amebocyte lysate reagent and measure BG more quickly and easily, it is attempted to measure BG by using an antibody recognizing BG (Patent Document 3 and 4, Non-Patent Documents 1, 2, and 4). However, the antibodies used in these methods have poor measurement sensitivity or are difficult to use in a so-called sandwich assay in which an antigen to be measured is sandwiched between two types of antibodies (immobilized antibody and labeled antibody). Additionally, no study has been conducted on a measurement system using an antibody recognizing BG and having a sensitivity equivalent to and a reactivity similar to the Limulus amebocyte lysate reagent, and an immunoassay method for measuring BG usable as a substitute for the Limulus amebocyte lysate reagent does not exist in the present circumstances.

### CITATION LIST

### PATENT LITERATURE

Patent Document 1: Japanese Patent No. 5089375
Patent Document 2: Japanese Patent No. 3553656
Patent Document 3: Japanese Laid-Open
Patent Publication No. 4-346791 Patent Document 4: WO 99/31510

### NON PATENT LITERATURE

Non-Patent Document 1: Use of beta-1,3-glucan-specific antibody to study the cyst wall of Pneumocystis carinii and effects of pneumocandin B0 analog L-733, 560. Antimicrobial Agents and Chemotherapy 1994 Oct; 38(10): 2258-2265
Non-Patent Document 2: Development of a two-site enzyme immunoassay based on monoclonal antibodies to measure airborne exposure to (1-3)-beta-D-glucan. Journal of Immunological Methods 2008 Aug 20; 337(1): 55-62
Non-Patent Document 3: Plasma (1-3) -beta-D-glucan Assay and Immunohistochemical staining of (1-3)-beta-D-glucan in the fungal cell walls using a novel horseshoe crab protein (T-GBP) that specifically binds to (1-3)-beta-D-glucan. Journal of Clinical Laboratory Analysis 11:104-199 (1997)
Non-Patent Document 4: The location of (1-3) -beta-glucans in the walls of pollen tubes of Nicotiana alata using a (1-3) -beta-glucan-specific monoclonal antibody. Planta (1991) 185: 1-8

### SUMMARY OF INVENTION

### TEHNICAL PROBLEM

As described above, the conventional technique of measuring BG using an antibody recognizing BG has many problems. Therefore, an immunoassay method for BG using an antibody has been desired that has the same detection sensitivity as the Limulus amebocyte lysate reagent, that exhibits the same reactivity as the Limulus amebocyte lysate reagent, and that can be used for performing a highly-sensitive sandwich assay.

### SOLUTION TO PROBLEM

The inventors analyzed in detail the reactivity of the Limulus amebocyte lysate reagent to various BGs. As a result, it was confirmed that the Limulus amebocyte lysate reagent reacts with (1→3)BG and (1→3)(1→6)BG among the BGs.

As described above, the structure of BG in biological samples is considered to be different depending on the causative species of deep mycosis, and particularly, (1->3)BG and (1->3)(1->6)BG exist in large number. In the structure of (1->3)(1->6)BG, a certain number of β(1→6) bonds are considered to be present in β(1→3) bonds. The present inventors conducted intensive studies for solving the problem and found out a possibility that an antibody recognizing long β(1→3) polymerization as an epitope cannot recognize(1→3)(1→6)BG since the β(1→6) bonds become an obstacle. The present inventors thought that, to have the same reactivity as the Limulus amebocyte lysate reagent and to detect the causative organisms of deep mycosis with high sensitivity, it is effective to produce an antibody recognizing short β(1→3) polymerization as an epitope, which has a possibility of recognizing both (1→3)BG and (1->3)(1->6)BG. The present inventors also thought that a highly-sensitive sandwich assay could be constructed by recognizing the short β(1→3) polymerization as an epitope.

Therefore, a plurality of antibodies recognizing short β(1→3) polymerization as an epitope was produced. By constructing a sandwich assay using these antibodies, the present inventors successfully construct a BG measurement system having a sensitivity equivalent to and a reactivity similar to the Limulus amebocyte lysate reagent, thereby completing the present invention.

Specifically, the present invention is as follows.
<1> An immunoassay method for β-D-glucan in a biological sample, comprising: assaying β-D-glucan in the biological sample by using two monoclonal antibodies binding to laminaritetraose.
<2> The immunoassay method according to <1>, wherein the biological sample is blood, plasma, or serum.
<3> The immunoassay method according to <1> or <2>, wherein the β-D-glucan in the biological sample is (1→3)-β-D-glucan and (1→3)(1→6)-β-D-glucan.
<4> The immunoassay method according to any one of <1> to <3>, wherein the concentration of β-D-glucan is 1 µg/mL or less.
<5> The immunoassay method according to any one of <1> to <4>, wherein ELISA is used.
<6> The immunoassay method according to any one of <1> to <5>, wherein the immunoassay method is a sandwich ELISA and wherein the two monoclonal antibodies binding to laminaritetraose are a first monoclonal antibody immobilized on a solid phase and a labeled second monoclonal antibody.
<7> The immunoassay method according to <6>, wherein the first monoclonal antibody immobilized on the solid phase is a mixture of multiple antibodies.
<8> The immunoassay method according to <6> or <7>, wherein the labelled second monoclonal antibody is a mixture of multiple antibodies.
<9> The immunoassay method according to any one of <1> to <8>, further comprising determining whether a subject has deep mycosis, and wherein a cutoff value of the determination is 20 pg/mL.
<10> The immunoassay method according to any one of <1> to <5>, wherein the monoclonal antibody has a competitive inhibitory action in an assay for β-D-glucan in a Limulus amebocyte lysate reagent.
<11> An assay kit for β-D-glucan in a biological sample comprising:
   (a) a solid phase on which a first monoclonal antibody binding to laminaritetraose is immobilized, wherein the assay further comprises
   (b) a second monoclonal antibody binding to laminaritetraose and labeled with a labeling substance.
<12> The assay kit according to <11>, wherein the biological sample is blood, plasma, or serum.
<13> The assay kit according to any one of <11> to <12>, wherein the β-D-glucan in the biological sample is (1→3)-β-D-glucan and (1→3)(1→6)-β-D-glucan.
<14> The assay kit according to any one of <11> to <13>, wherein the concentration of β-D-glucan is 1 µg/mL or less.
<15> The assay kit according to any one of <11> to <14>, wherein the kit is used for determining whether a subject has deep mycosis, and wherein a cutoff value of the determination is 20 pg/mL.

### ADVANTAGEOUS EFFECTS OF INVENTION

According to the present invention, an immunoassay method for BG in a biological sample having a sensitivity equivalent to and a reactivity similar to the Limulus amebocyte lysate reagent can be performed. According to the present invention, a kit for immunoassay for BG in a biological sample having the same sensitivity and the same reactivity as the Limulus amebocyte lysate reagent can be provided.

### BRIEF DESCRIPTION OF DRAWINGS

[Fig. 1] Fig. 1 is a schematic showing a merit of an antibody recognizing short β(1→3) polymerization as an epitope in detecting a clinical specimen.
[Fig. 2] Fig. 2 is a graph showing results of competitive ELISA for screening antibodies recognizing short β(1→3) polymerization as an epitope.
[Fig. 3] Fig. 3 is a graph showing results of epitope analysis of 86202R antibody.
[Fig. 4] Fig. 4 is a graph showing results of epitope analysis of 86207 antibody.
[Fig. 5] Fig. 5 is a graph showing results of sandwich ELISA using 86202R antibody and 86207 antibody with various BGs.
[Fig. 6] Fig. 6 is a graph showing results of analysis of the various BGs using a commercially available reagent 1.
[Fig. 7] Fig. 7 is a graph showing results of analysis of various BGs using a commercially available reagent 2.
[Fig. 8] Fig. 8 is a graph showing results of study on a lower limit of detection of an immunoassay method for BG of the present invention.
[Fig. 9] Fig. 9 is a graph showing a correlation between the immunoassay method for BG of the present invention and the commercially available reagent 1.
[Fig. 10] Fig. 10 is a graph showing a correlation between the immunoassay method for BG of the present invention and the commercially available reagent 2.
[Fig. 11] Fig. 11 is a graph showing results of a competitive experiment of measurement with the commercially available reagent 2 using an antibody used in the immunoassay method for BG of the present invention.
[Fig. 12] Fig. 12 is a graph showing results of determination of the presence or absence of fungal infection in a healthy person plasma sample using a cutoff value (20 pg/mL) of the Limulus amebocyte lysate reagent.

### DESCRIPTION OF EMBODIMENTS

### [1] Immunoassay Method for β-D-Glucan in Biological Sample

### (Biological Sample)

Examples of a "biological sample" in the present invention mainly include solid tissues and body fluids derived from living bodies (organisms), and body fluids are preferably used. The biological sample in the present invention is more preferably blood, serum, plasma, urine, saliva, sputum, tear fluid, otorrhea, or prostatic fluid, further preferably blood, serum, or plasma, further preferably blood, serum, or plasma of a subject suspected of having deep mycosis, most preferably blood, serum, or plasma of a subject suspected of having deep mycosis caused by causative fungi of the genus Candida and/or the genus Aspergillus. Examples of the living body or the subject include humans or animals (e.g., monkeys, dogs, cats, mice, guinea pigs, rats, hamsters, horses, bovines, pigs, birds, and fish), and are preferably humans.

In the immunoassay method of the present invention, a biological sample is pretreated as needed. Examples of the pretreatment include an alkaline treatment and a heat treatment, or these treatments can be combined. A treatment time, pH of an alkaline solution, a type of an alkaline reagent, a treatment temperature, etc. may appropriately be set depending on the type etc. of the biological sample.

### (β-D-glucan)

In this description, "β-D-glucan" and "BG" mean a glucose polymer in which multiple glucose molecules are bound by β-bonds. BG is a cell wall constituent component of fungi, and measurement of BG in body fluid has been used for auxiliary diagnosis of deep mycosis infections. Examples of BG can include (1→3)-β-D-glucan, (1→3)(1→6)-β-D-glucan, (1→3)(1→4)-β-D-glucan (β-glucan derived from barley, lichenin, etc.). In the immunoassay method of the present invention, β-D-glucan in the biological sample to be detected is preferably (1→3)-β-D-glucan and/or (1→3)(1→6)-β-D-glucan.

### ((1→3)-β-D-Glucan)

In this description, "(1→3)-β-D-glucan" and "BG" mean glucan in which carbon at the 1-position of glucose and carbon at the 3-position of other glucose are bonded in a binding form of β-type. BG has a unique structure called a triple helix structure.

Examples of include pachyman, curdlan, laminaritetraose, paramylon, carboxymethylpachyman, carboxymethylcurdlan, (1->3)BG present in the cell walls of Aspergillus fungi, and (1->3)BG present in the cell walls of Candida fungi.

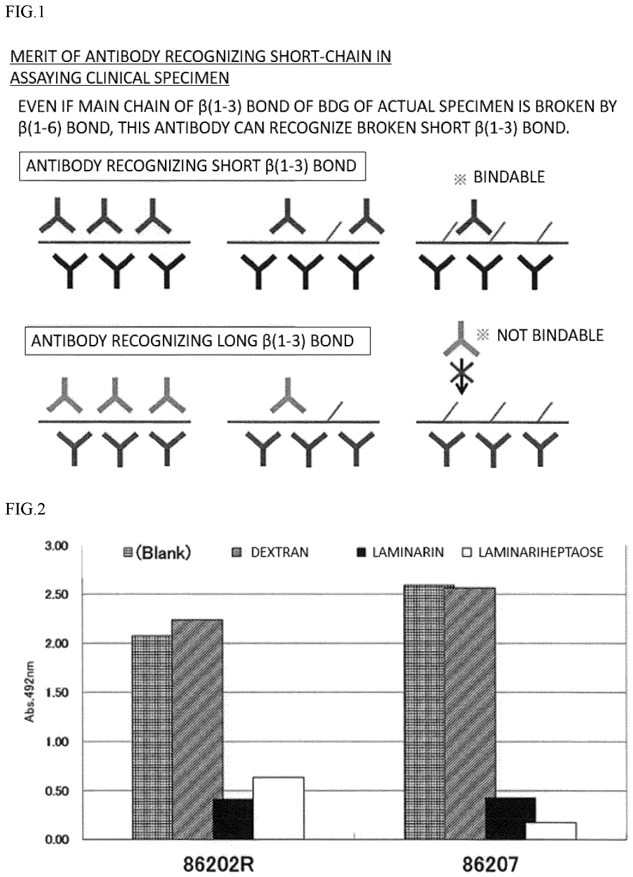

In this description, (1->3)BG having glucose bound to the outer side chain of the triple helix structure in the (1→6) form in the structure of (1->3)BG is referred to as (1→3)(1→6)-β-D-glucan or (1->3)(1->6)BG. Examples of (1→3)(1→6)-β-D-glucan include laminarin, sizofiran, (1->3)(1->6)BG present in the cell walls of Aspergillus fungi, and (1->3)(1->6)BG present in the cell walls of Candida fungi. The (1->3)(1->6)BG has a structure in which glucose is bound to the outer side chain of the triple helix structure in the (1→6) form, and an antibody recognizing long β(1→3) polymerization probably cannot recognize the (1->3)(1->6)BG since the glucose bound to the outer side chain of the triple helix structure in the (1→6) form inhibits the binding of the antibody (Fig. 1).

A monoclonal antibody used in the present invention preferably reacts with none of the structures of (1→4)-β-D-glucan, (1→6)-β-D-glucan, (1→6)-α-D-glucan, (1→4)(1→6)-α-D-glucan, (1→6)(1→6)-α-D-glucan, and (1→4)-α-D-glucan.

Specifically, a monoclonal antibody used in the present invention preferably does not react with any of the followings: mannan and carboxymethyl cellulose, which are (1→4)-β-D-glucans; palstan, which is (1→6)-β-D-glucan; dextran, which is (1→6)-α-D-glucan; glycogen and amylopectin, which are (1→4)(1→6)-α-D-glucans; pullulan, which is (1→6)(1→6)-α-D-glucan; and amylose, which is (1→4)-α-D-glucan.

As used herein, the "deep mycosis" means a condition in which a fungus entering a deep part of the body such as lungs, liver, kidneys, or brain causes infection. The deep mycosis mainly occurs in patients having undergone organ transplantation and receiving immunosuppressive drugs. Examples of the causative fungi of deep mycosis include Aspergillus fungi and Candida fungi, and the immunoassay method of the present invention can effectively analyze BG present in the cell walls of these fungi.

### (Monoclonal antibodies Binding to (1→3)-β-D-Glucan Having Degree of Polymerization of 4)

The monoclonal antibodies used in the present invention are monoclonal antibodies binding to (1→3)-β-D-glucan having a degree of polymerization of 4. "Binding to (1→3)-β-D-glucan having a degree of polymerization of 4" means recognizing a β(1→3) bond having a degree of polymerization of 4 as an epitope. In this description, "assaying β-D-glucan in a biological sample by using a monoclonal antibody binding to (1→3)-β-D-glucan having a degree of polymerization of 4" means bringing a biological sample into contact with the monoclonal antibodies and comparing an obtained measured value with a cutoff value.

The degree of polymerization of "4" means that four glucose molecules are polymerized.

An anti-(1→3)BG antibody used in the present invention preferably reacts with BG, preferably (1->3)BG and/or (1->3)(1->6)BG, preferably at a free antigen concentration of 1 µg/mL or less, more preferably a free antigen concentration of 100 ng/mL or less.

Specific examples of the monoclonal antibody used in the present invention include a 86202R antibody, which is the monoclonal antibody produced from the hybridoma NITE BP-02723 and a 86207 antibody, which is the monoclonal antibody produced from the hybridoma NITE BP-02724.

Although "reacting" with (1→3)-β-D-glucan having a degree of polymerization of 4, "recognizing" (1→3)-β-D-glucan having a degree of polymerization of 4, and "binding" to (1→3)-β-D-glucan having a degree of polymerization of 4 are synonymously used in this description, they must be construed in the broadest sense without being limited to these exemplifications. Whether an antibody "reacts" with an antigen (compound) can be confirmed by an antigen-solid-phased ELISA method, a competitive ELISA method, a sandwich ELISA method, etc, as well as a method using the principle of surface plasmon resonance (SPR method) etc. The SPR method can be performed by using devices, sensors, and reagents commercially available under the name of Biacore (registered trademark).

Stating that the antibody used in the present invention "does not react with" a certain compound means that the antibody used in the present invention does not substantially react with a certain compound, while stating "not substantially reacting" means that enhanced reactivity of the antibody used in the present invention is not recognized when Biacore (registered trademark) T100 or T200 is used for immobilizing and measuring the antibody of the present invention based on the SPR method, for example. Specifically, this means that the reactivity between the antibody and the compound is not significantly different from the reactivity of a control (with no compound added). Obviously, it can be confirmed that the antibody "does not substantially react" with the compound by a method or means well known to those skilled in the art, in addition to the SPR method.

The monoclonal antibodies used in the immunoassay method of the present invention includes a fragment having a function of the monoclonal antibodies as long as the effects of the present invention can be obtained. Examples of the fragment include a functional fragment containing the Fab portion of the monoclonal antibodies obtained by enzymatic digestion of the monoclonal antibodies, a functional fragment containing the Fab portion of the monoclonal antibodies prepared by gene recombination, and a functional fragment containing scFv prepared by a phage display method, etc.

The antibodies used in the immunoassay method of the present invention can be produced by dissolving (1->3)BG having a degree of polymerization of 4 such as laminariheptaose as an antigen (immunogen) in a solvent such as phosphate buffered saline and administering this solution to immunize an animal. A hybridoma producing an antibody binding to (1->3)BG having a degree of polymerization of 4 may be screened by using (1->3)BG having a degree of polymerization of 5 or more such as laminariheptaose as an antigen (immunogen). The immunization may be performed by using an emulsion after adding an appropriate adjuvant to the solution as required. The adjuvant may be a widely used adjuvant, such as water-in-oil emulsion, water-in-oil-in-water emulsion, oil-in-water emulsion, liposome, or aluminum hydroxide gel as well as a protein or peptidic substance derived from biological components. For example, Freund's incomplete or complete adjuvant can preferably be used. Although not particularly limited, it is desired that the administration route, administered dose, and administration time of the adjuvant are appropriately selected such that a desired immune response can be enhanced in an animal to be immunized by the antigen.

Although not particularly limited, the type of the animal used for the immunization is preferably a mammal and can be a mouse, rat, bovine, rabbit, goat, sheep, and alpaca, and a mouse or rat is more preferable. The animal may be immunized in accordance with a common technique and, for example, the immunization can be achieved by subcutaneously, intracutaneously, intravenously, or intraperitoneally injecting the animal with a solution of an antigen, preferably a mixture with the adjuvant. Since an immune response is generally different depending on the type and strain of the animal to be immunized, it is desirable that an immunization schedule is appropriately set depending on the animal to be used. The antigen administration is preferably repeated several times after initial immunization.

Although the following operations are continuously performed so as to obtain a monoclonal antibody, the operation is not limited thereto, and a method of producing the monoclonal antibodies itself is well known in the art and is widely used, so that the antibodies used in the immunoassay method of the present invention can easily be produced by using the antigen described above (see, e.g., Antibodies, A Laboratory Manual (Cold Spring Harbor Laboratory Press, (1988), Chapter 6)).

After final immunization, a hybridoma can be produced by extracting spleen or lymph node cells, which are antibody-producing cells, from an immunized animal and by fusing the cells with a myeloma-derived cell line having high proliferative ability. Cells having high antibody-producing ability (quantitative and qualitative) are preferably used for the cell fusion, and the myeloma-derived cell line is preferably compatible with the animal from which the antibody-producing cells to be fused are derived. The cell fusion can be performed in accordance with a method known in the art, and a polyethylene glycol method, a method using Sendai virus, or a method utilizing electric current can be employed. The obtained hybridoma can be proliferated in accordance with conditions widely used in the art, and the desired hybridoma can be selected while confirming a property of a produced antibody. The hybridoma can be cloned by a well-known method such as a limiting dilution method and a soft agar method.

The hybridoma can efficiently be selected at the selection stage in consideration of the conditions under which the produced antibody is actually used in the measurement. For example, an antibody obtained by immunizing an animal is reacted with (1->3)BG having a degree of polymerization of 4 immobilized on a solid phase in the presence of a compound for which cross-reactivity is desired to be confirmed, and the reactivity can be compared with that in the absence of the compound for which cross-reactivity is desired to be confirmed so as to more efficiently select the hybridoma producing a desired antibody. Alternatively, an antibody obtained by immunizing an animal is reacted with (1->3)BG having a degree of polymerization of 4 immobilized on a solid phase in the presence of a biological sample-derived component, and the reactivity can be compared with that in the absence of the biological sample-derived component so as to more efficiently select the hybridoma producing a desired antibody.

After a cloning step, the binding ability of the produced antibody to (1->3)BG can be assayed by using a method such as an ELISA method, an RIA method, and a fluorescent antibody method so as to confirm whether the selected hybridoma produces a monoclonal antibody having a desired property.

A monoclonal antibody having a desired property can be produced by the mass cultivation of the hybridoma selected as described above. Although a method of mass cultivation is not particularly limited, examples thereof can include a method of producing the monoclonal antibody in culture media by cultivating the hybridoma in appropriate culture media and a method of producing the antibody in abdominal dropsy by injecting the hybridoma into the abdominal cavity of a mammal for proliferation. The monoclonal antibody can be purified by appropriately combining the methods for purifying an antibody from the antiserum described above, for example, DEAE anion exchange chromatography, affinity chromatography, an ammonium sulphate fractionation method, a PEG fractionation method, and an ethanol fractionation method.

The monoclonal antibodies used in the immunoassay method of the present invention can be a whole antibody molecule as well as a fragment of an antibody having an antigen-antibody reaction activity. The antibodies can be obtained through an immunization step of an animal as described above or obtained by a gene recombination technique or can be a chimeric antibody. The fragment of the antibodies is preferably a functional fragment; examples thereof include F(ab')₂, Fab', scFv, etc.; and these fragments can be produced by processing the antibodies obtained as described above with a proteolytic enzyme (e.g., pepsin or papain), or by cloning of DNA of the antibodies and expression in a culture system using Escherichia coli or yeast.

The antibodies used in the immunoassay method of the present invention can be used as an immobilized (solid-phased) antibody immobilized on an insoluble carrier or as a labeled antibody labeled with a labeling substance well known to those skilled in the art described later. For example, the immobilized antibody can be produced by causing an insoluble carrier to physically adsorb or chemically bind to a monoclonal antibody (a suitable spacer may exist therebetween). The insoluble carrier can be made of a polymer base material such as a polystyrene resin, an inorganic base material such as glass, and a polysaccharide base material such as cellulose and agarose, and the shape thereof is not particularly limited and can be selected from any shapes such as a plate shape (e.g., microplate and membrane), a bead or particle shape (e.g., latex particles), and a tubular shape (e.g., test tube).

By using a labeled antibody (secondary antibody) that can bind to the antibodies used in the immunoassay method of the present invention, an amount of the antibodies bound to BG can be measured, and BG in a biological sample can thereby be detected. The BG in the biological sample is preferably and/or (1->3)(1->6)BG. Examples of the labeling substance for producing the labeled antibody include enzymes, fluorescent substances, chemiluminescent substances, biotin, avidin, radioisotopes, colloidal gold particles, or colored latex. A method for binding the labeling substance and the antibodies can be a method such as a glutaraldehyde method, a maleimide method, a pyridyl disulfide method, or a periodic acid method, which can be used by those skilled in the art, and the types of the immobilized and labeled antibodies and the methods for producing the antibodies are not limited to these examples. For example, when an enzyme such as horseradish peroxidase (HRP) or alkali phosphatase (ALP) is used as the labeling substance, the enzyme activity can be measured by using a specific substrate of the enzyme (e.g., O-phenylenediamine (OPD) or 3,3',5,5'-tetramethylbenzidine (TMB) when the enzyme is HRP, p-nitrophenyl phosphate in the case of ALP), and when biotin is used as the labeling substance, at least avidin or enzyme-modified avidin is typically reacted therewith. In the immunoassay method of the present invention, biotin or HRP is preferably used as the labeling substance.

In this description, an "insoluble carrier" may be represented as a "solid phase", and physically or chemically supporting an antigen or antibody with an insoluble carrier or the supporting state may be represented as "immobilizing", "immobilized", or "solid phased". The term "assay", "detection", or "measurement" must be construed in the broadest sense, including the existence proof and/or the quantitation of BG and must not be construed in a limited manner in any sense.

### (Immunoassay Method)

Examples of the immunoassay method of the present invention include but not limited to ELISA, enzyme immunoassay, an immunohistochemical staining method, a surface plasmon resonance method, latex agglutination immunoassay, chemiluminescence immunoassay, electrochemiluminescence immunoassay, a fluorescent antibody method, radioimmunoassay, an immunoprecipitation method, a Western Blot method, immunochromatography, high performance liquid chromatography (HPLC), etc. It is preferable to use ELISA as the immunoassay method of the present invention. Among ELISAs, a sandwich ELISA using a first monoclonal antibody immobilized on a solid phase and a labeled second monoclonal antibody is preferable.

In this case, the 86207 antibody can be used as the first monoclonal antibody, and the 86202R antibody can be used as the second monoclonal antibody. The same antibody may be used as the first monoclonal antibody and the second monoclonal antibody. The antibody immobilized on the solid phase or the labeled antibody may be a mixture of multiple antibodies. Biotin or HRP is preferably used as the labeling substance for the labeled antibody.

### (Diagnosis, Diagnostic Assistance, and Treatment of Deep Mycosis)

Based on an analysis result of the immunoassay method of the present invention, a diagnosis can be made on whether the subject has deep mycosis, or the diagnosis can be assisted. The sensitivity of the conventional immunoassay method using an antibody is insufficient, and a patient with deep mycosis in the early stage of the disease may have a negative result. Early detection and early treatment are important for deep mycosis. By using the highly-sensitive immunoassay method of the present invention, early detection and early treatment of deep mycosis can be achieved.

After performing the immunoassay method of the present invention, if necessary, based on a result of a step of analyzing BG in a biological sample, another method for analysis of deep mycosis may be implemented for the patient, and/or a drug for the treatment of deep mycosis may be administered to the patient.

The immunoassay method of the present invention can include a step of detecting 4 pg/mL or more of BG contained in a biological sample (e.g., blood, serum, or plasma, preferably plasma) and/or a step of diagnosing a subject corresponding to the collected biological sample as having or being suspected of having deep mycosis, based on the result of the detection step, or a step of assisting the diagnosis. A cutoff value can appropriately be set depending on a type of the biological sample or a type of the immunoassay method. For example, the immunoassay method of the present invention can include a step of detecting 6 pg/mL or more, 7 pg/mL or more, 8 pg/mL or more, 9 pg/mL or more, 10 pg/mL or more, 11 pg/mL or more, 20 pg/mL or more, 50 pg/mL or more, 80 pg/mL or more, 100 pg/mL or more, 150 pg/mL or more, 200 pg/mL or more, 250 pg/mL or more, 300 pg/mL or more , 400 pg/mL or more, 500 pg/mL or more, 1 ng/mL or more, 5 ng/mL or more, or 10 ng/mL or more of BG contained in a biological sample (e.g., blood, serum, or plasma, preferably plasma), and/or a step of diagnosing a subject corresponding to the collected biological sample as having or being suspected of having deep mycosis, based on the result of the detection step, or a step of assisting the diagnosis.

In a commercially available Limulus amebocyte lysate reagent, and/or (1->3)(1->6)BG in the sample bind to the G-factor. This binding initiates a cascade so that (1->3)BG and/or (1->3)(1->6)BG is analyzed. The monoclonal antibodies used in the immunoassay method of the present invention preferably recognizes a G-factor binding site in (1->3)BG and/or (1->3)(1->6)BG.

The monoclonal antibodies used in the immunoassay method of the present invention preferably have a competitive inhibitory action in the analysis of β-D-glucan, preferably (1->3)BG and/or (1->3)(1->6)BG with the Limulus amebocyte lysate reagent. In this case, the competitive inhibitory action specifically means that the binding of the monoclonal antibodies to β-D-glucan inhibits the binding of β-D-glucan to the G-factor, and as a result and consequently inhibits the progress of reaction cascade of the Limulus amebocyte lysate reagent.

When the monoclonal antibodies used in the immunoassay method of the present invention has the competitive inhibitory action in the analysis of β-D-glucan with the Limulus amebocyte lysate reagent, the concentration of the monoclonal antibodies in a reaction system is 0.1 µg/mL to 1000 µg/mL, preferably 1 µg/mL to 100 µg/mL.

### [2] β-D-glucan Analysis Kit in Biological Sample

The kit provided by the present invention includes (a) a solid phase on which a first monoclonal antibody binding to (1->3)-P-D-glucan having a degree of polymerization of 4 is immobilized and preferably includes (b) a second monoclonal antibody binding to (1->3)-P-D-glucan with a degree of polymerization of 4 and labeled with a labeling substance. The solid phase having the first monoclonal antibody immobilized thereon captures BG in the biological sample and forms a BG-antibody complex. The second anti-BG monoclonal antibody labeled with the labeling substance reacts with this BG-antibody complex to form a sandwich. The BG in the sample can be measured by measuring an amount of the labeling substance by a method corresponding to the labeling substance. For specific methods for constructing the kit, such as a method for immobilizing the first monoclonal antibody on the solid phase and a method for labeling the second monoclonal antibody with a labeling substance, the methods described in this description and the methods well known to those skilled in the art can be used without limitation.

The first monoclonal antibody and the second monoclonal antibody are not particularly limited as long as the antibodies are monoclonal antibodies binding to (1→3)-β-D-glucan having a degree of polymerization of 4. For example, the 86207 antibody can be used as the first monoclonal antibody, and the 86202R antibody can be used as the second monoclonal antibody.

For the labeling substance, for example, a labeling substance known to those skilled in the art such as a fluorescent substance, a chemiluminescent substance, biotin, and avidin can be used. A method for binding the labeling substance and the antibody can appropriately be selected from known binding methods depending on the labeling substance and antibody to be used and can be, for example, a method such as a glutaraldehyde method, a maleimide method, a pyridyl disulfide method, or a periodic acid method. Biotin or HRP is preferably used as the labeling substance.

The kit of the present invention can also include an instruction manual etc. The kit may include any constituent elements, such as a buffer, a stabilizer, and a reaction vessel.

The kit of the present invention can detect 4 pg/mL or more of BG contained in a biological sample (e.g., blood, serum, or plasma, preferably plasma), and based on this result, the subject corresponding to the collected biological sample can be diagnosed as having or being suspected of having deep mycosis. The cutoff value can appropriately be set depending on a type of the biological sample etc. For example, the kit of the present invention can detect 6 pg/mL or more, 7 pg/mL or more, 8 pg/mL or more, 9 pg/mL or more, 10 pg/mL or more, 11 pg/mL or more, 15 pg/mL or more, 20 pg/mL or more, 24.9 pg/mL or more, 25 pg/mL or more, 30 pg/mL or more, 50 pg/mL or more, 80 pg/mL or more, 100 pg/mL, 150 pg/mL or more, 200 pg/mL or more, 250 pg/mL or more, 300 pg/mL or more, 400 pg/mL or more, 500 pg/mL or more, 1 ng/mL or more, 5 ng/mL or more, or 10 ng/mL or more of BG contained in a biological sample (e.g., blood, serum, or plasma, preferably plasma), and based on this result, the subject corresponding to the collected biological sample can be diagnosed as having or being suspected of having deep mycosis.

The present invention will hereinafter specifically be described with examples; however, these examples do not limit the scope of the present invention.

### Examples

### [Test Example 1] Method for Producing Monoclonal Antibody Used in the Present Invention.

### 1. Preparation of Immunizing Antigen

Laminariheptaose (manufactured by Seikagaku Biobusiness Corporation) is having a structure with 7 glucose molecules polymerized in a linear shape and was used as an immunizing antigen. A laminariheptaose-transferrin conjugate was prepared by the same preparation method as described in Non-Patent Document 1 and used as an immunizing antigen.

### 2. Production of Hybridoma and Collection of Antibody

The whole amount of a suspension composed of 100 µL of solution of the prepared laminariheptaose-transferase conjugate (1 mg/mL), 0.25 mL of 0.1 M PBP (pH 7.2), and 0.25 mL of Freund's adjuvant (complete adjuvant or incomplete adjuvant) was administered subcutaneously to the back of, or intraperitoneally to, each of a BALB/c mouse (female) and an F344/Jc1 rat (female) 3 to 6 times in total. An administration interval was 2 weeks, and Freund's complete adjuvant was used for the first administration, while Freund's incomplete adjuvant was used for the latter four administrations. One week after the fifth administration, the abdominal cavities of the mouse and the rat were opened to remove their spleens, and single cells were obtained by pipetting.

These splenocytes were washed twice with PRMI1640 medium with no serum added and were mixed with separately cultured and washed mouse myeloma cells (X-63-Ag8-6.5.3) at a ratio of 1×10⁷ myeloma cells to 5×10⁷ splenocytes, and the mixture was centrifuged to remove the supernatant. The sediment was well dissolved, polyethylene glycol 1540 (1 mL) serving as a fusion accelerator was slowly added at 37 °C for 1 minute, and the mixture was further stirred for 1 minute for fusion. After these fused cells (hybridomas) were suspended in 10 mL of RPMI1640 medium supplemented with fetal bovine serum and were centrifuged, the residue was seeded on a single 96-well culture plate and cultured in a 5% CO₂ incubator at 37 °C for 1 week. After culturing in HAT medium at 37 °C for 1 week, only hybridomas were selectively collected.

### 3. Antigen-Solid-Phased ELISA (Primary Screening)

Primary screening of hybridomas was performed by the method described below. Laminarin was used as the antigen. Laminarin is (1->3)(1->6)BG and has a structure in which glucose molecules is bound to the outer side chain of the triple helix structure in the (1→6) form. It is considered that the antibody recognizing laminarin may recognize the short β(1→3) binding between glucose molecules bound in the (1→6) form.
1) A laminarin solution was dispensed into a plate (50 µL/well) and allowed to stand at room temperature for 2 hours or at 4 °C overnight.
2) After washing 3 times (400 µL/well), a blocking solution was dispensed (100 µL/well) and allowed to stand at room temperature for 1 hour.
3) After washing 3 times (400 µL/well), a dilution series of a culture supernatant was dispensed (50 µL/well) and allowed to stand at room temperature for 1 hour.
4) After washing 3 times (400 µL/well), an HRP-labeled goat anti-mouse IgG polyclonal antibody was dispensed (50 µL/well) and allowed to stand at room temperature for 1 hour.
5) After washing 3 times (400 µL/well), an OPD coloring solution was dispensed (50 µL/well) and reacted at room temperature for 10 minutes.
6) A stop solution was dispensed (50 µL/well) to stop the reaction, and measurement was performed with a plate reader.
7) Fifty hybridomas producing antibodies highly reactive with laminarin were selected and used in the following competitive ELISA.

### 4. Competitive ELISA (Secondary Screening)

Secondary screening of hybridomas (confirmation of reactivity of established candidate strains) was performed by the method described below.
1) A laminarin solution was dispensed into an ELISA plate (50 µL/well) and allowed to stand at room temperature for 2 hours or at 4 °C overnight.
2) After washing 3 times (400 µL/well), the blocking solution was dispensed (100 µL/well) and allowed to stand at room temperature for 1 hour.
3) After washing 3 times (400 µL/well), sugar chains (dextran, laminarin, or laminariheptaose) were dispensed (25 µL/well). Subsequently, a culture supernatant was dispensed (25 µL/well) and allowed to stand at room temperature for 1 hour.
4) After washing 3 times (400 µL/well), an HRP-labeled goat anti-mouse IgG polyclonal antibody was dispensed (50 µl/well) and allowed to stand at room temperature for 1 hour.
5) After washing 3 times (400 µL/well), an OPD coloring solution was dispensed (50 µL/well) and reacted at room temperature for 10 minutes.
6) A stop solution was dispensed (50 µL/well) to stop the reaction, and measurement was performed with a plate reader.
7) The results are shown in Fig. 2. The 86207 (from mouse) and the 86202R (from rat) were selected since both caused competition when laminarin and laminariheptaose were used as free antigens.

### 5. Antibody Purification Procedure

The monoclonal antibody was purified by using the two obtained hybridomas. Ascites was extracted from mice in which hybridoma cells were intraperitoneally injected and cryopreserved at -80 °C. An ascites dissolving liquid was added to the ascites for thawing in a water bath, and centrifugation was performed to collect a supernatant.

To the recovered supernatant, 50 % ammonium sulfate was added. Centrifugation was performed again, and a redissolving liquid was added to a precipitate fraction to dissolve the precipitate. The dissolved solution was centrifuged, and a supernatant was collected. The supernatant was dialyzed overnight at 4 °C by using a dialysate (20 mM NaPi pH 7.5, 100 mM NaCl, 1 mM EDTA 2Na) in 100 times the amount of the supernatant. For dialysis, Slide-A-Lyzer Diarysis cassette 10k (manufactured by Thermo Fisher Scientific) was used.

On the next day, the protein G column Hi Trap Protein G HP 5 mL (manufactured by GE Healthcare) was connected to the low-pressure chromatography system AKTA Prime plus (manufactured by GE Healthcare), and the dialyzed sample solution was separated and purified. The protein G column was preliminarily equilibrated with a column equilibrium solution. After adding the sample solution to the column, the antibody bound to the column was eluted with a pH 4 eluting solution. After elution at pH 4, elution was further performed with a pH 3 eluting solution. The sample solutions eluted with the pH 4 eluting solution and the pH 3 eluting solution were each dialyzed by an antibody preservation solution (PBS). The dialysis was performed twice at 4 °C with 100 times the amount of the sample solution. After the dialysis, sodium azide was added at a final concentration of 0.09 %, and the mixture was divided into 400 µL aliquots and stored at -80 °C. Two antibodies (the 86202R antibody and the 86207 antibody) were obtained by the procedure described above. The antibody sample eluted at pH 4 was used for the following measurements.

### [Example 2] Verification of Reactivity of Antibody to (1->3)BGs having multiple degree of polymerizations

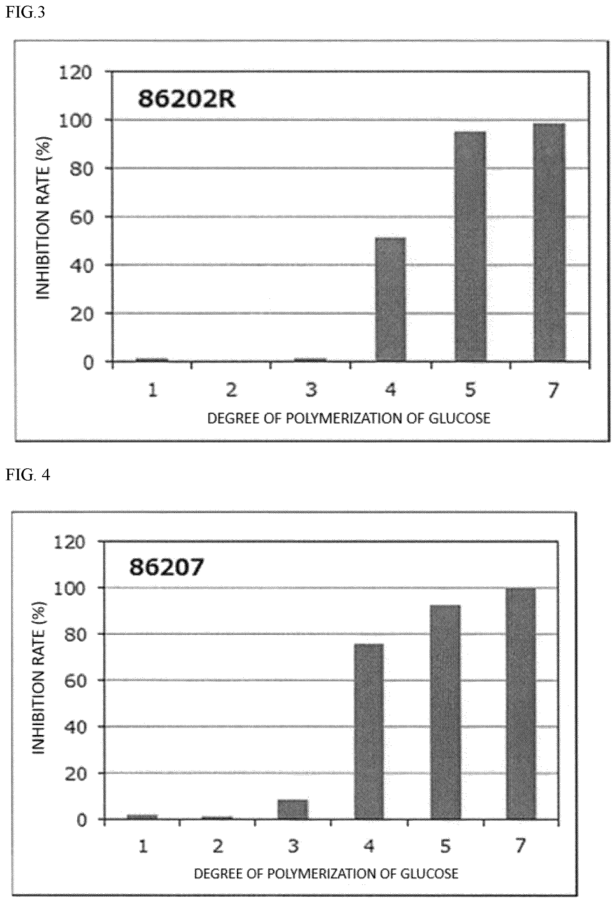

Competitive ELISA was performed by using (1→3)BGs having a degree of polymerization of 1 to 7, and the reactivity of the 86202R antibody and the 86207 antibody to the (1->3)BG having each degree of polymerization was verified. For the (1→3)BGs having a degree of polymerization of 1 to 7, glucose (degree of polymerization is 1), laminaribiose (degree of polymerization is 2), laminaritriose (degree of polymerization is 3), laminaritetraose (degree of polymerization is 4), laminarpentaose (degree of polymerization is 5), and laminariheptaose (degree of polymerization is 7) were used. Laminarihexaose (degree of polymerization is 6) was not used for verification since it was difficult to obtain raw materials. Glucose was purchased from FUJIFILM Wako Pure Chemical Corporation, and the other (1→3)BGs were purchased from Santa Cruz Biotechnology.
1) Laminarin was dispensed into an ELISA plate (1 µg/ml in PBS, 50 µL/well) and allowed to stand at 4 °C overnight.
2) After washing 3 times (400 µL/well), a blocking solution was dispensed (100 µL/well) and allowed to stand at room temperature for 1 hour.
3) After washing 3 times (400 µL/well), each of the (1→3)BGs was dispensed (25 µL/well, the concentration of each of the (1->3)BGs was 100 µg/ml). Subsequently, the 86202R antibody solution or the 86207 antibody solution was dispensed (25 µL/well) and allowed to stand at room temperature for 1 hour.
4) After washing 3 times (400 µL/well), an HRP-labeled goat anti-rat IgG polyclonal antibody or HRP-labeled goat anti-mouse IgG polyclonal antibody was dispensed (50 µl/well) and allowed to stand at room temperature for 1 hour.
5) After washing 3 times (400 µL/well), an OPD coloring solution was dispensed (50 µL/well) and reacted at room temperature for 10 minutes.
6) A stop solution was dispensed (50 µL/well) to stop the reaction, and measurement was performed with a plate reader.
7) The result of the 86202R antibody is shown in Fig. 3, and the result of the 86207 antibody is shown in Fig. 4.

The 86202R antibody recognized the (1→3)BGs having a degree of polymerization is of 4 to 7. Therefore, the antibody was considered as an antibody recognizing a β(1→3) bond having a degree of polymerization of 4.

The 86207 antibody recognized the (1->3)BGs having a degree of polymerization of 4 to 7. Therefore, the antibody was considered as an antibody recognizing a β(1→3) bond having a degree of polymerization of 4. Considering a difference in reactivity between the two antibodies, the two antibodies probably have slightly different recognition sites.

### [Example 3] Confirmation of Reactivity to BG Having Different Binding Form

Sandwich ELISA was performed by using the 86207 antibody as the solid-phase antibody and the 86202R antibody as the HRP-labeled antibody (liquid-phase antibody), and the reactivity to various BGs was compared with Limulus amebocyte lysate reagents. Regarding the Limulus amebocyte lysate reagents, β-Glucan Test Wako (FUJIFILM Wako Pure Chemical Corporation) (hereinafter "commercially available reagent 1") and Fungitec (registered trademark) G Test ES "Nissui" (Nissui Pharmaceutical Co., Ltd.) (hereinafter "commercially available reagent 2") were used, and experiments were conducted in accordance with attached protocols. The antigens used are as follows.

**[Table 1]**

| Antigen | Binding form |
|---|---|
| Carboxymethylation-modified curdlan | β(1-3) |
| Carboxymethylation-modified pachyman | β(1-3) |
| Laminarin | (1-3)β(1-6) |
| Lichenan | β(1-3)β(1-4) |
| Carboxymethylation-modified cellulose | β(1-4) |
| Glycogen | α(1-4)α(1-6) |
| Pullulan | α(1-6)α(1-6) |
| Mannan | β(1-4) |
| GALACTOMANNAN | Mβ(1-4)Mβ(1-4)Gβ(1-4) |

The procedure for sandwich ELISA will be described below.
1) The solid-phase antibody (5 µg/mL, 100 µL, diluted with PBS) was dispensed into each well of a 96-well plate (nunc immunoplate, part number 442404, Thermo Fisher Scientific) and allowed to stand at room temperature for 2 hours or at 4 °C overnight.
2) After removing the liquid from each well, 200 µL of PBST was dispensed twice into each plate well by using an 8-channel Pipetman (400 µL in total). The added PBST was removed, and these operations were defined as one wash operation. This operation was performed three times (=washing with 400 µL of PBST three times; the same procedure was performed for washing in the following operations).
3) After washing, 200 µL of a blocking solution was dispensed into each well and allowed to stand at room temperature for 1 hour or more.
4) After discarding the blocking solution, each serially diluted BG was added and reacted for 60 minutes.
5) After the reaction, the removal of the liquid in each well was followed by washing with 400 µL of PBST three times.
6) The HRP-labeled antibody (0.5 µg/mL, 100 µL, diluted with the blocking solution) was dispensed and reacted for 60 minutes.
7) After the reaction, the removal of the liquid in each well was followed by washing with 400 µL, of PBST three times.
8) Into each well, 100 µL of a coloring solution was dispensed and reacted at room temperature for 10 minutes.
9) Into each well, 100 µL of a reaction stop solution was added.
10) The absorbance at 492 nm was measured with a microplate reader (Multican FC, thermo scientific).

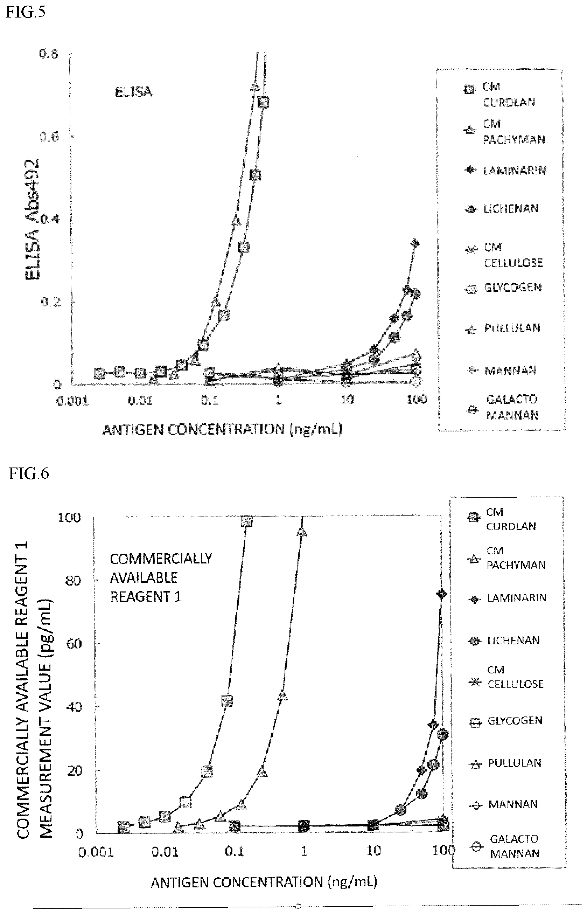

The results are shown in Figs. 5 to 7. In sandwich ELISA using an antibody recognizing BG, when (1->3)BG was used as an antigen, a reaction was observed from an antigen concentration of about 0.1 ng/mL. When (1->3)(1->6)BG was used as an antigen, a reaction was observed from an antigen concentration of about 10 ng/mL (Fig. 5). No reaction occurred with the other BGs.

In the case of the commercially available reagent 1, when was used as an antigen, a reaction was observed from an antigen concentration of about 0.1 ng/mL. When (1->3)(1->6)BG was used as an antigen, a reaction was observed from an antigen concentration of about 10 ng/mL (Fig. 6). No reaction occurred with the other BGs.

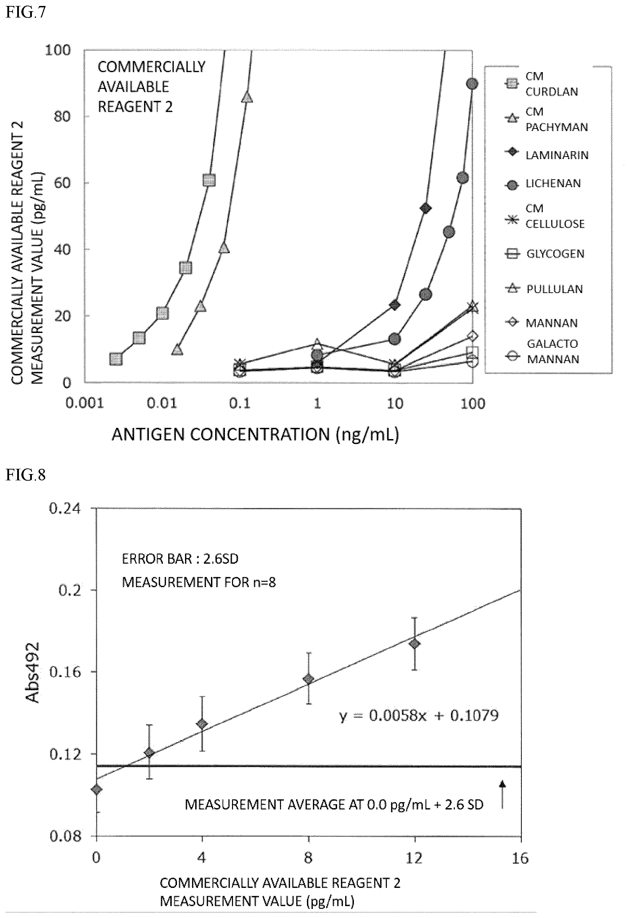

In the case of the commercially available reagent 2, when was used as an antigen, a reaction was observed from an antigen concentration of about 0.01 ng/mL. When (1->3)(1->6)BG was used as an antigen, a reaction was observed from an antigen concentration of about 1 ng/mL (Fig. 7). No reaction occurred with the other BGs.

Therefore, it was demonstrated that the sandwich ELISA using an antibody recognizing BG has the same reactivity to (1->3)BG and (1->3)(1->6)BG as the Limulus amebocyte lysate reagent manufactured by Wako Pure Chemical Industries, Ltd. (commercially available reagent 1) and the Limulus amebocyte lysate reagent (commercially available reagent 2) manufactured by Nissui Pharmaceutical Co., Ltd.

### [Example 4] Examination of Lower Limit of Detection ofBG in Biological Sample

In Example 4, the detection lower limit of sandwich ELISA was examined by using a biological sample. The 86207 antibody was used as the solid-phase antibody, and the HRP-labeled 86202R antibody was used as the liquid-phase antibody.

### 4-1. Pretreatment with Alkali

A biological sample (human plasma containing 24 pg/mL BG derived from fungus, measured by the commercially available reagent 2) was diluted in stages with PBST to prepare a dilution series. To 176 µL of the specimen, 309.6 µL of an alkaline treatment solution (150 mM KOH: potassium hydroxide) was added and incubated at 37 °C for 15 minutes. Subsequently, 394.4 µL of 1 M Tris/HCl (pH 7.5) was added to neutralize the alkaline treatment solution (880 µL in total, 5-fold dilution of sample, for 8 measurements). These specimens were used as alkali pretreated specimens.

### 4-2. HRP Labeling of Antibody

HRP labeling of the 86202R antibody was performed by using an HRP labeling kit (Peroxidase Labeling Kit-SH, LK09, manufactured by Dojindo Laboratories). Protein quantification after labeling was performed by a BCA method (kit name: Micro BCA protein assay kit, manufactured by Thermo Scientific, part number: 23235).

### 4-3. Sandwich ELISA

The 86207 antibody (5 µg/mL, 100 µL, diluted with PBS) for the solid phase was dispensed into each well of a 96-well plate (nunc immunoplate, part number 442404, manufactured by Thermo Fisher Scientific) and allowed to stand overnight at 4 °C.

After removing liquid from each well, 200 µL of PBST was dispensed into each plate well twice by using an 8-channel Pipetman (400 µL in total). The added PBST was removed, and these operations were defined as one wash operation. This operation was performed three times (washing with 400 µL of PBST three times; the same procedure was performed for washing in the following operations).

After washing, 200 µL of a blocking solution was dispensed into each well and allowed to stand at room temperature for 1 hour or more.

After discarding the blocking solution, 100 µL of each of the pretreated specimens was added to each well and reacted for 90 minutes.

After the reaction, the removal of the liquid in each well was followed by washing with 400 µL of PBST three times.

The HRP-labeled 86202R antibody (0.5 µg/mL, 100 µL, diluted with the blocking solution) prepared in 3-2 was dispensed into each well and reacted for 30 minutes.

After the reaction, the removal of the liquid in each well was followed by washing with 400 µL of PBST three times.

Into each well, 100 µL of a coloring solution was dispensed and reacted at room temperature for 10 minutes.

Into each well, 100 µL of a reaction stop solution was added.

The absorbance at 492 nm was measured with a microplate reader (Multiskan FC, manufactured by thermo Fisher Scientific). Each sample was measured for n=8.

### 4-4. result

The results are shown in Table 2 and Fig. 8. The absorbance in Table 2 represents an average value of measurement for n=8, and SD represents a standard deviation.

**[Table 2]**

| Actual density (pg/mL) | Absorbance (Average value of n=8) | SD | 2.6SD | Average value ±2.6 SD |
|---|---|---|---|---|
| 0.0 | 0.103 | 0.0043 | 0.0112 | 0.1142 |
| 2.0 | 0.121 | 0.0050 | 0.0130 | 0.1080 |
| 4.0 | 0.135 | 0.0051 | 0.0133 | 0.1217 |
| 8.0 | 0.157 | 0.0048 | 0.0125 | 0.1445 |
| 12.0 | 0.174 | 0.0049 | 0.0127 | 0.1613 |

The value (0. 1142) obtained by adding 2.6 SD of the sample to the average value of the absorbance of the sample having an actual concentration of 0.0 pg/mL is smaller than the value (0.1217) obtained by subtracting 2.6 SD of the sample from the average absorbance of the sample of 4.0 pg/mL, and therefore, it was found that even when BG derived from fungus contained in the actual sample is 4.0 pg/mL (the measurement value of the commercially available reagent 2), BG can be detected in ELISA serving as an embodiment of the immunoassay method of the present invention. The cutoff value of the commercially available reagent 2 is 20 pg/mL, and this result indicated that ELISA serving as an embodiment of the immunoassay method of the present invention has the sensitivity equivalent to that of the Limulus amebocyte lysate reagent.

### [Example 5] Examination of correlation with Limulus amebocyte lysate reagent

In Example 5, the correlation between the measurement value of sandwich ELISA and the measurement values of the commercially available reagents 1 and 2 was examined by using a human plasma sample (n=39). The 86207 antibody was used as the solid-phase antibody, and the HRP-labeled 86202R antibody was used as the liquid-phase antibody.

### 5-1. Pretreatment with Alkali

In correlation comparison with the commercially available reagent 1, a specimen was subjected to the following pretreatment. After adding 14.7 µL of a human plasma specimen and 29.3 µL of PBST, 77.4 µL of an alkali pretreatment solution (150 mM KOH: potassium hydroxide) was added and incubated at 37 °C for 15 minutes. Subsequently, 98.6 µL of 1 M Tris/HCl (pH 7.5) was added to the alkali pretreatment solution to neutralize the alkali pretreatment solution (220 µL in total; 15-fold dilution of the specimen; pH after neutralization was less than 7.9).

In correlation comparison with the commercially available reagent 2, a specimen was subj ected to the following pretreatment. To 44 µL of a human plasma specimen, 77.4 µL of an alkali pretreatment solution (150 mM KOH: potassium hydroxide) was added and incubated at 37 °C for 15 minutes. Subsequently, 98.6 µL of 1 M Tris/HCl (pH 7.5) was added to the alkali pretreatment solution to neutralize the alkali pretreatment solution (220 µL in total; 5-fold dilution of the specimen; pH after neutralization was less than 7.9). This was used as an alkali-pretreated specimen.

### 5-2. HRP Labeling of Antibody

HRP labeling of the antibody was performed in the same procedure as Example 3.

### 5-3. Sandwich ELISA

Sandwich ELISA was performed in the same procedure as Example 3. Each sample was measured for n=2, and an average value was used as a measured value.

### 5-4. Limulus Amebocyte Lysate Reagent

BG was measured in accordance with the protocol described in respective package inserts of commercially available reagents 1 and 2.

### 5-5. Result

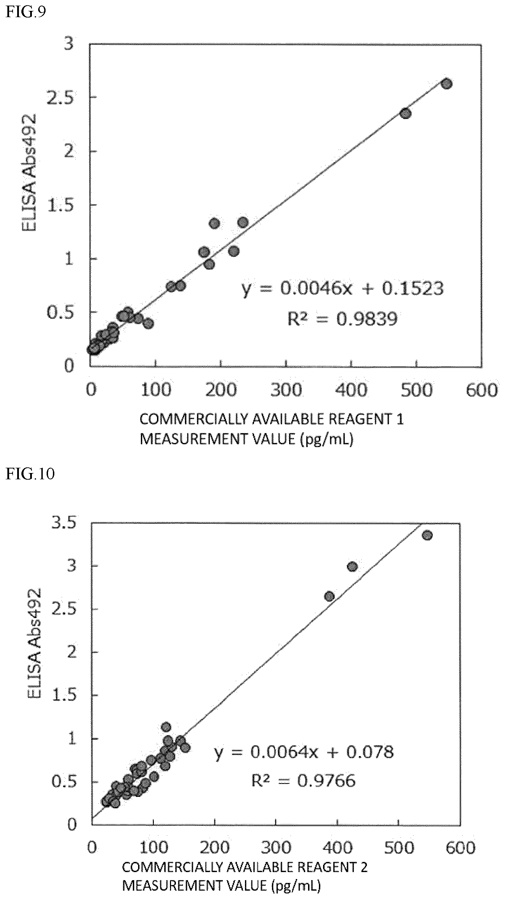

Fig. 9 shows a correlation between the measured value of the commercially available reagent 1 and the absorbance according to ELISA. Fig. 10 shows a correlation between the measured value of the commercially available reagent 2 and the absorbance according to ELISA.

The absorbance measurement value according to sandwich ELISA of an embodiment of the immunoassay method of the present invention showed a correlation with both measurement values according to the Limulus amebocyte lysate reagents of the commercially available reagents 1 and 2.

### [Example 6] Test for Competition with Limulus Amebocyte Lysate Reagent

To examine whether the 86207 antibody and the 86202R antibody used in Example 5 inhibit a cascade reaction of the Limulus amebocyte lysate reagent of the commercially available reagent 2, a competitive assay of these two monoclonal antibodies and the commercially available reagent 2 was performed.

The experiment was conducted with slight modification added to the attached protocol of the commercially available reagent 2. A measurement sample was PBS-Tween containing 1 % BSA in which CM-pachyman was dissolved. The measurement sample was incubated at 25 °C for 60 minutes in the presence or absence of 10 µg/mL antibody. Subsequently, 200 µL of distilled water was added to 50 µL of the measurement sample (a pretreatment solution of the commercially available reagent 2 was not used so as to prevent denaturation of the antibody). After incubating at 37 °C for 10 minutes, 50 µL of the measurement sample was added to 300 µL of the commercially available reagent 2. The cascade reaction of the commercially available reagent 2 was measured at 37 °C for 30 minutes with an ES analyzer (manufactured by Nissui Pharmaceutical Co., Ltd.).

An anti-surfactant protein D antibody (manufactured by Abcam) was used for a control experiment of a mouse IgG antibody. Each experiment was performed 3 times, and an average value was calculated.

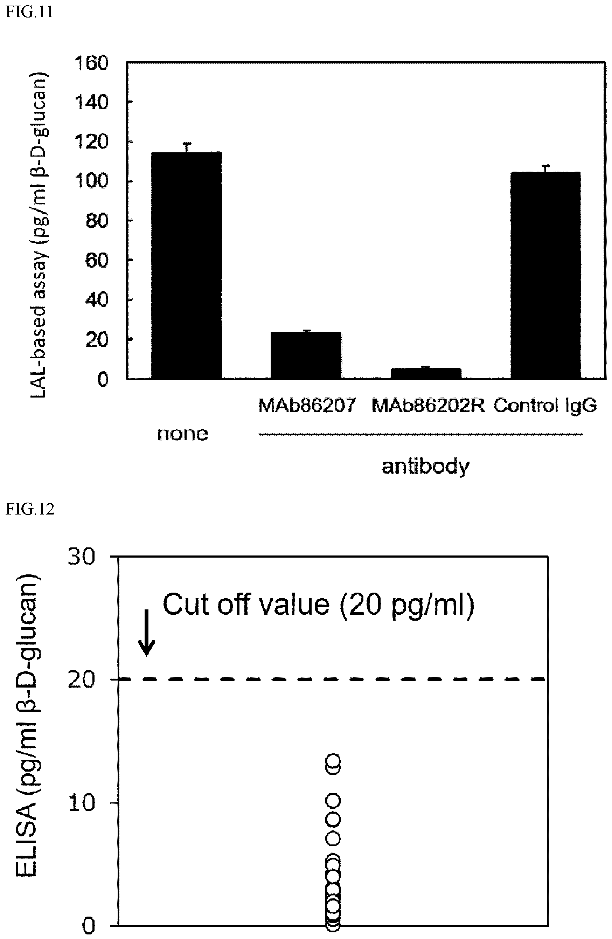

The results are shown in Fig. 11. Both the 86207 antibody and the 86202R antibody suppressed the activity of the commercially available reagent 2 by 80.0 % and 95.5 %, respectively. The control IgG antibody did not inhibit the activity of the commercially available reagent 2 as compared to that in the absence of the antibody. The results demonstrate that both the 86207 antibody and the 86202R antibody recognize a structure in BG activating the G-factor in the reaction cascade in the commercially available reagent 2.

### [Example 7] Measurement of Healthy Person Plasma Sample Using Cut-Off Value (20 pg/mL) of Limulus Amebocyte Lysate Reagent

An experiment was conducted by using 32 specimens of healthy person plasma samples so as to confirm whether fungal infection can be diagnosed in the same way as the Limulus amebocyte lysate reagent by using ELISA using the 86207 antibody and the 86202R antibody. The experimental procedure was the same as Example 5 (correlation comparison with the commercially available reagent 2). The results are shown in Fig. 12.

The measurement values of the specimens were all less than the cutoff value of the Limulus amebocyte lysate reagent of 20 pg/mL and had the median of 3.6 pg/mL. Therefore, it was demonstrated that fungal infection can be diagnosed in the same way as the Limulus amebocyte lysate reagent by using ELISA using the 86207 antibody and the 86202R antibody.

### INDUSTRIAL APPLICABILITY

The present invention enables an immunoassay method of BG in a biological sample having a sensitivity equivalent to and a reactivity similar to the Limulus amebocyte lysate reagent. The present invention can provide a kit for immunoassay for BG in a biological sample having a sensitivity equivalent to and a reactivity similar to the Limulus amebocyte lysate reagent.

### ACCESSION NUMBER

### [Reference to Deposited Biological Material]

(1) Hybridoma 86202R producing the antibody number 86202R
   i) Name and address of depository institution at which the biological materials were deposited.
      NITE Patent Microorganisms Depositary, National Institute of Technology and Evaluation
      2-5-8 Kazusakamatari, Kisarazu-shi, Chiba 2920818, Japan
   ii) Date of biological material deposit in the depository institution in i).
      May 17, 2018
   iii) Accession number for the deposition assigned by the depository institution in i).
      NITE BP-02723
(2) Hybridoma 86207 producing the antibody number 86207
   i) Name and address of depository institution at which the biological materials were deposited.
      NITE Patent Microorganisms Depositary, National Institute of Technology and Evaluation
      2-5-8 Kazusakamatari, Kisarazu-shi, Chiba 2920818, Japan
   ii) Date of biological material deposit in the depository institution in i).
      May 17, 2018
   iii) Accession number for the deposition assigned by the depository institution in i).
      NITE BP-02724

## Claims

1. An immunoassay method for β-D-glucan in a biological sample, comprising: assaying β-D-glucan in the biological sample by using two monoclonal antibodies binding to laminaritetraose.

2. The immunoassay method according to claim 1, wherein the biological sample is blood, plasma, or serum.

3. The immunoassay method according to claim 1 or 2, wherein the β-D-glucan in the biological sample is (1→3)-β-D-glucan and (1→3)(1→6)-β-D-glucan.

4. The immunoassay method according to any one of claims 1 to 3, wherein the concentration of β-D-glucan is 1 µg/mL or less.

5. The immunoassay method according to any one of claims 1 to 4, wherein ELISA is used.

6. The immunoassay method according to any one of claims 1 to 5, wherein the immunoassay method is a sandwich ELISA and wherein the two monoclonal antibodies binding to laminaritetraose are a first monoclonal antibody immobilized on a solid phase and a labeled second monoclonal antibody.

7. The immunoassay method according to claim 6, wherein the first monoclonal antibody immobilized on the solid phase is a mixture of multiple antibodies.

8. The immunoassay method according to claim 6 or 7, wherein the labeled second monoclonal antibody is a mixture of multiple antibodies.

9. The immunoassay method according to any one of claims 1 to 8, further comprising determining whether a subject has deep mycosis, and wherein a cutoff value of the determination is 20 pg/mL.

10. The immunoassay method according to any one of claims 1 to 5, wherein the monoclonal antibody has a competitive inhibitory action in an assay for β-D-glucan in a Limulus amebocyte lysate reagent.

11. An assay kit for β-D-glucan in a biological sample comprising:
(a) a solid phase on which a first monoclonal antibody binding to laminaritetraose is immobilized, wherein the assay further comprises
(b) a second monoclonal antibody binding to laminaritetraose and labeled with a labeling substance.

12. The assay kit according to claim 11, wherein the biological sample is blood, plasma, or serum.

13. The assay kit according to any one of claims 11 to 12, wherein the β-D-glucan in the biological sample is (1→3)-β-D-glucan and (1→3)(1→6)-β-D-glucan.

14. The assay kit according to any one of claims 11 to 13, wherein the concentration of β-D-glucan is 1 µg/mL or less.

15. The assay kit according to any one of claims 11 to 14, wherein the kit is used for determining whether a subject has deep mycosis, and wherein a cutoff value of the determination is 20 pg/mL.

## Patentansprüche

1. Immunoassay-Verfahren für β-D-Glucan in einer biologischen Probe, umfassend:
Testen von β-D-Glucan in der biologischen Probe unter Verwendung von zwei monoklonalen Antikörpern, die an Laminaritetraose binden.

2. Immunoassay-Verfahren gemäß Anspruch 1, wobei die biologische Probe Blut, Plasma oder Serum ist.

3. Immunoassay-Verfahren gemäß Anspruch 1 oder 2, wobei das β-D-Glucan in der biologischen Probe (1->3)-β-D-Glucan und (1->3)(1->6)-β-D-Glucan ist.

4. Immunoassay-Verfahren gemäß irgendeinem der Ansprüche 1 bis 3, wobei die Konzentration von β-D-Glucan 1 µg/ml oder weniger ist.

5. Immunoassay-Verfahren gemäß irgendeinem der Ansprüche 1 bis 4, wobei ELISA verwendet wird.

6. Immunoassay-Verfahren gemäß irgendeinem der Ansprüche 1 bis 5, wobei das Immunoassay-Verfahren ein Sandwich-ELISA ist und wobei die zwei an Laminaritetraose bindenden monoklonalen Antikörper ein erster auf einer festen Phase immobilisierter monoklonaler Antikörper und ein markierter zweiter monoklonaler Antikörper sind.

7. Immunoassay-Verfahren gemäß Anspruch 6, wobei der erste auf der festen Phase immobilisierte monoklonale Antikörper eine Mischung aus mehreren Antikörpern ist.

8. Immunoassay-Verfahren gemäß Anspruch 6 oder 7, wobei der markierte zweite monoklonale Antikörper eine Mischung aus mehreren Antikörpern ist.

9. Immunoassay-Verfahren gemäß irgendeinem der Ansprüche 1 bis 8, ferner umfassend das Bestimmen, ob eine Person eine tiefe Mykose hat, und wobei ein Grenzwert der Bestimmung 20 pg/ml ist.

10. Immunoassay-Verfahren gemäß irgendeinem der Ansprüche 1 bis 5, wobei der monoklonale Antikörper eine kompetitive Inhibitionswirkung in einem Assay für β-D-Glucan in einem Limulus-Amöbozytenlysatreagenz hat.

11. Testkit für β-D-Glucan in einer biologischen Probe, umfassend:
(a) eine feste Phase, auf der ein erster monoklonaler Antikörper, der an Laminaritetraose bindet, immobilisiert ist, wobei der Test ferner umfasst
(b) einen zweiten monoklonalen Antikörper, der an Laminaritetraose bindet und mit einer Markierungssubstanz markiert ist.

12. Testkit gemäß Anspruch 11, wobei die biologische Probe Blut, Plasma oder Serum ist.

13. Testkit gemäß irgendeinem der Ansprüche 11 bis 12, wobei das β-D-Glucan in der biologischen Probe (1->3)-β-D-Glucan und (1->3) (1->6)-β-D-Glucan ist.

14. Testkit gemäß irgendeinem der Ansprüche 11 bis 13, wobei die Konzentration von β-D-Glucan 1 µg/ml oder weniger ist.

15. Testkit gemäß irgendeinem der Ansprüche 11 bis 14, wobei das Kit zur Bestimmung verwendet wird, ob eine Person eine tiefe Mykose hat, und wobei ein Grenzwert der Bestimmung 20 pg/ml ist.

## Revendications

1. Procédé de dosage immunologique pour le β-D-glucane dans un échantillon biologique comprenant : le dosage du β-D-glucane dans l'échantillon biologique en utilisant deux anticorps monoclonaux se liant au laminaritétraose.

2. Procédé de dosage immunologique selon la revendication 1, dans lequel l'échantillon biologique est du sang, du plasma ou du sérum.

3. Procédé de dosage immunologique selon la revendication 1 ou la revendication 2, dans lequel le β-D-glucane dans l'échantillon biologique est du (1→3)-β-D-glucane et du (1→3)(1→6)-β-D-glucane.

4. Procédé de dosage immunologique selon l'une quelconque des revendications 1 à 3, dans lequel la concentration de β-D-glucane est de 1 µg/ml ou moins.

5. Procédé de dosage immunologique selon l'une quelconque des revendications 1 à 4, dans lequel une ELISA est utilisée.

6. Procédé de dosage immunologique selon l'une quelconque des revendications 1 à 5, dans lequel le procédé de dosage immunologique est une ELISA prise en sandwich et dans lequel les deux anticorps monoclonaux se liant au laminaritétraose sont un premier anticorps monoclonal immobilisé sur une phase solide et un second anticorps monoclonal marqué.

7. Procédé de dosage immunologique selon la revendication 6, dans lequel le premier anticorps monoclonal immobilisé sur la phase solide est un mélange de multiples anticorps.

8. Procédé de dosage immunologique selon la revendication 6 ou la revendication 7, dans lequel le second anticorps monoclonal marqué est un mélange de multiples anticorps.

9. Procédé de dosage immunologique selon l'une quelconque des revendications 1 à 8, comprenant en outre le fait de déterminer si un sujet présente une mycose profonde, et dans lequel une valeur de coupure de la détermination est de 20 pg/ml.

10. Procédé de dosage immunologique selon l'une quelconque des revendications 1 à 5, dans lequel l'anticorps monoclonal présente une action inhibitrice compétitive dans un dosage pour le β-D-glucane dans un réactif de lysat d'amibocyte de Limulus.

11. Kit de dosage pour le β-D-glucane dans un échantillon biologique comprenant :
(a) une phase solide sur laquelle un premier anticorps monoclonal se liant au laminaritétraose est immobilisé, dans lequel le dosage comprend en outre
(b) un second anticorps monoclonal se liant au laminaritétraose et marqué avec une substance de marquage.

12. Kit de dosage selon la revendication 11, dans lequel l'échantillon biologique est du sang, du plasma ou du sérum.

13. Kit de dosage selon l'une quelconque des revendications 11 à 12, dans lequel le β-D-glucane dans l'échantillon biologique est du (1→3)-β-D-glucane et du (1→3)(1→6)-β-D-glucane.

14. Kit de dosage selon l'une quelconque des revendications 11 à 13, dans lequel la concentration de β-D-glucane est de 1 µg/ml ou moins.

15. Kit de dosage selon l'une quelconque des revendications 11 à 14, dans lequel le kit est utilisé pour déterminer si un sujet présente une mycose profonde, et dans lequel une valeur de coupure de la détermination est de 20 pg/ml.
